# EUROPEAN PATENT APPLICATION

(11) **EP 3 228 304 A1**
(43) Date of publication of application: **11.10.2017**
(21) Application number: 17164493.3
(22) Date of filing: 03.04.2017
(51) Int. Cl.: A61K 8/41, A61Q 1/10, A61Q 5/06, A61K 8/92, A61K 8/06

(54) **COSMETIC COMPOSITION**

(30) Priority: 04.04.2016 US 201662317798 P; 03.02.2017 US 201715423730
(71) Applicant: Mascara Plus S.r.l., 20065 Milano (IT)
(72) Inventor: CHANT, David, 20065 Milano (IT); KLAIWONG, Tummawat, 20065 Milano (IT)
(74) Representative: Gerbino, Angelo

(57) **Abstract**

The present disclosure relates to cosmetic compositions that do not contain triethanolamine, and methods for preparing the same. The compositions of the present disclosure comprise a wax-in-water emulsion and a water-in-oil co-emulsifier, the combination of which produces compositions with similar texture, consistency, and/or feel to those obtained using triethanolamine.

## Description

### BACKGROUND

The use of triethanol amine in cosmetic products, for example mascara, is becoming increasingly regulated. As such, a cosmetic composition with an insignificant amount of triethanol amine, or a composition that is completely free of triethanol amine, is desirable. However, because triethanol amine can impart one or more desirable cosmetic properties, for example texture, consistency, and/or feel, designing a composition that retains these properties while containing only an insignificant amount of triethanol amine, or no triethanol amine, is a challenge. Triethanol amine can breakdown and thus create Nitrosamines(Ndela), which if ingested orally in laboratory rats, are known to cause cancer. While mascara is not ingested, the market is nevertheless asking for Triethanol amine free mascara formulations. The European limit for Nitrosamines (Ndela) are 50 ppb, while in southern Germany 10 ppb, so it would be preferable to eliminate the triethanol amine.

### SUMMARY

The following presents a simplified summary of one or more aspects of the present disclosure in order to provide a basic understanding of such aspects. This summary is not an extensive overview of all contemplated aspects and is intended to neither identify key or critical elements of all aspects nor delineate the scope of any or all aspects. Its purpose is to present some concepts of one or more aspects in a simplified form as a prelude to the more detailed description that is presented later.

In some embodiments, the present disclosure is directed to a cosmetic composition as claimed comprising a wax-in-water emulsion and a water-in-oil co-emulsifier present at 1-20% by weight, wherein the composition does not contain triethanolamine.

In some embodiments, the present disclosure is directed to a method of preparing a cosmetic composition as claimed, the method comprising admixing a wax-in-water emulsion with a water-in oil co-emulsifier, wherein the water-in-oil co-emulsifier is present at 1-20% by weight, to obtain a cosmetic composition that does not contain triethanolamine. All aspects described with respect to the composition apply with equal force to the method.

These and other aspects of the invention will become more fully understood upon a review of the detailed description, which follows.

### DETAILED DESCRIPTION

The composition of the present invention can be formulated as a variety of cosmetic products. For example, the composition can be formulated as hair dye, conditioner, shampoo, a combination of one or more of hair dye, conditioner, and shampoo, mascara, or eyebrow pen liquid. Importantly, the composition can also find use in areas other than cosmetics, for example, the composition could be a component of household products, dyes, pharmaceuticals, resins, paints, coatings, lubricants, and other products.

It has been discovered that the combination of a wax-in-water emulsion along with a water-in-oil co-emulsifier produces a composition having similar properties in terms of texture, consistency, and/or feel, as that obtained by using triethanol amine. It is believed that the use of the wax-in-water emulsion along with a water-in-oil co-emulsifier keeps the wax cells apart such that a network of wax cells is not formed in the product, which thereby allows for a product that is not too hard and has sticky consistency.

Suitable water-in-oil co-emulsifiers include, but are not limited to one or more of, polyglyceryl-3-rice branate, cetearyl alcohol, polyglyceryl-10 stearate, polyglyceryl-6 stearate, hydroxypropyl guar, cetearyl alcohol, glyceryl stearate, PEG-30 dipolyhydroxystearate, PEG-40 stearate, cetearet-20, 165-PEG-100 stearate, and glyceyl stearate. In some aspects, the water-in-oil co-emulsifier may be present in the composition at a level of 1-20% by weight.

In addition to the wax-in-water emulsion and the water-in-oil co-emulsifier, the composition can contain one or more cosmetically acceptable ingredients. Several types of cosmetically acceptable ingredients, along with examples of the various types, are discussed below. A person of ordinary skill in the art would readily understand which cosmetically acceptable ingredients to use depending on the desired product. Importantly, while several types of exemplary cosmetically acceptable ingredients are discussed herein, the composition does not necessarily contain type of cosmetically acceptable ingredient discussed. Further, the composition may contain other types of ingredients other than those discussed. Although specific examples of the various types of cosmetically acceptable ingredients are discussed, other cosmetically acceptable ingredients can also be used. The amounts of such ingredients can range from 0.0001% to 99.9% by weight or volume of the composition, or any integer or range in between as disclosed in other sections of this specification, which are incorporated into this paragraph by reference.

The composition can contain one or more waxes, for example, joba wax, rice wax, carnauba wax, candelilla wax, beeswax, lanolin wax, orange peel wax, Chinese insect wax, ouricury wax, cork fiber wax, sugar cane wax, Japan wax, sumach wax, montan wax, microcrystalline waxes, paraffins, polyethylene waxes, methicones, such as methicone and C1-C45 alkyl, phenyl, and mixed C1-C45 alkyl and phenyl methicones, dimethicones, such as dimethicone, C1-C45 alkyl, and mixed C1-C45 alkyl and phenyl dimethicones, and simethicones. In some aspects, the one or more waxes may be present in the composition at a level of 5-30 % by weight.

The composition can contain also one or more gums, for example acacia gums, such as acacia Senegal gum, gum arabic, gum ghatti, gum karay, gum tragacanth, guar gum, guar hydroxypropyl ammonium chloride, xanthan gum, gellan gum, and mixtures thereof. In some aspects, the one or more gums may be present in the composition at a level of 1-10 % by weight.

The composition can contain one or more cellulose polymers, such as cellulose, sodium or potassium carboxymethyl cellulose, hydroxyethyl cellulose, ethyl cellulose, hydroxypropyl cellulose, sulfated cellulose, hydroxypropylmethyl cellulose, microcrystalline cellulose, and mixtures thereof. In some aspects, the one or more cellulose polymers may be present in the composition at a level of 1-10 % by weight.

The composition can also contain one or more volatile oils, such as paraffins or isoparaffin having from 8 to 16 carbon atoms, isododecane, isodecane, propylene glycol n-butyl ether, ethyl 3-ethoxy propionate, propylglycol methylether acetates, decamethyltetrasiloxane, octatrimethicone, hexatrimethicone, decamethylcyclopeta siloxane, octamethylcyclotetrasiloxane, dodecamethylcyclohexyl siloxane, polydimethyl siloxande (PDMS), and decamethyltetra siloxane. In some aspects, the one or more volatile oils may be present in the composition at a level of 5-25 % by weight.

The composition can contain water. In some aspects, water may be present in the composition at a level of 45-60 % by weight.

The composition can contain one or more low molecular weight alcohols, for example, ethyl alcohol, propyl alchol, such as n-propyl or isopropyl alcohol, or butyl alcohol, such as n-butyl, isobutyl, sec-butyl or t-butyl alcohol. In some aspects, the one or more low molecular weight alcohols may be denatured. In some aspects, the one or more low molecular weight alcohols may be present in the composition at a level of 0.1-5 % by weight.

The composition can contain one or more humectants, for example, polyhydric alcohols, glycerol, glycerin, C1 to C4 alkane glycols, such as butylene propylene, and ethylene glycol, alkylene polyols, hyaluronic acid, urea, sorbitol, 2-pyrrolidone-5-carboxylate salts, such as sodium or potassium salts, collagen, dibutylphthalate, and gelatin. In some aspects, the one or more humectants may be present in the composition at a level of 0.5-5 % by weight.

The composition can contain one or more fillers, pigments, and colorants, for example, iron oxides, talc, mica, magnesium carbonate, calcium carbonate, magnesium silicate, aluminum magnesium silicate, silica, polyethylene, (meth)acrylate, polystyrene, silk, crystalline cellulose, starch, bismuth oxide, zinc oxide, salts or lakes of aluminun, barium, or calcium, organic dyes, such as FD&C dyes or lakes thereof, and carbon black. The one or more pigments, colorants, or fillers can be treated with one or more hydrophilic polymers, for example, polyethylene glycols and polyquaterniums. The one or more pigments or colorants can be present in solution or as dispersed particles. The one or more pigments or colorants can also be adapted to produce a pearlescent effect. In some aspects, the one or more fillers, pigments, and colorants may be present in the composition at a level of 0.1-5 % by weight.

The composition can include one or more preservatives, for example parabens, such as methyl, ethyl, propyl, dimethyl, or butyl paraben, phenoxy ethanol, ethylhexyl glycerin, sodium dehydroacetate, potassim sorbate, imidazoleidinyl urea, capryl glycol, hexylene glycol, pheoxy ethanol, p-hydroxy benzoate and esters thereof. The one or more preservatives can be present in a amount sufficient to prevent or inhibit the growth of microbes. In some aspects, the one or more preservatives may be present in the composition at a level of 0.01-5 % by weight.

The composition can also contain one or more film forming agents, for example, waxes, such as the waxes discussed above, polyolefins, ethylene vinyl acetate, dimethocone gum, polyterpenes, silionce resins, such as trimethylsiloxy silicate, vinylpyrrolidone/eicosene copolymer, vinylpyrrolidone/hexadecene copolymer, vinylpyrrolidone/vinyl acetate copolymer, vinylpyrrolidone/(alk)acrylate copolymer, for example copolymers of vinyl pyrrolidone with one or more of ethacrylate, methacrylate, acrylate, acrylic acid, vinylpyrrolidone/styrene copolymer, ethyl cellulose, propyl cellulose, polyalkylenes, such as polymers or copolymers of C2 to C20 polyalkylenes or polybutylene, polyvinylpyrrolidone, and partially or wholely butylated polyvinylpyrrolidone. The one or more film forming agents can be present in an amount sufficient to ensure that the cosmetic composition forms an acceptable film, for example, on the skin or on one or more keratinous fibers, such as the hair, and often the eyelashes or eyebrows. In some aspects, the one or more film forming agents may be present in the composition at a level of 0.5-2 % by weight.

The composition can include one or more structuring agents, for example, stearic acid, palmitic acid, stearyl alcohol, cetyl alcohol, polyethylene glycol ethers of stearyl alcohol, polyethylene glycol ethers of cetyl alcohol, behenyl alcohol, steareth-2 (i.e., a polyethylene glycol ether of stearyl alcohol with an average of about 2 ethylene oxide units per stearyl alcohol unit), acrylic acid/ethyl acrylate copolymers, carbomers, carboxyvinyl polymers, water-soluble polymers of polyalkenyl polyether polymer of acrylic acid crosslinked with from 0.75% to 2.0% of polyallyl sucrose or polyallyl pentaerythritol, lic acid crosslinked with about 1% of polyallyl ether of sucrose, acrylates/10-30 alkyl acrylate cross-polymers, waxes, such as the waxes discussed above, Fischer-Tropsch waxes, silicone waxes, C24-C45 methicones, saturated polyglucosides, trihydroxystearin, hydroxy cellulose, hycroxymethyl cellulose, ethy cellulose, and methyl cellulose. The one or more structuring agents can be present in an amount sufficient to ensure that the cosmetic composition, when applied to the skin or to one or more keratinous fibers, such as the hair, and often the eyelashes or eyebrows, maintains a sufficient structure to obtain a cosmetically desirable effect. In some aspects, the one or more structuring agents may be present in the composition at a level of 0.1-8 % by weight.

The composition can include one or more fragrances. The one or more fragrances may be present in an amount sufficient to impart a pleasing or commercially acceptable odor. In some aspects, the one or more fragrances may be present in the composition at a level of 0.01-5 % by weight.

The composition may also include one or more fibers, such as polyester fibers, rayon fibers, nylon fibers, and polyamide fibers. When the composition is designed for application to one or more keratinous fibers, for example the eyebrows or eyelashes, the one or more fibers may be present in an amount sufficient to achieve a fiber-lengthening effect, such as a lash-lengthening effect. In some aspects, the one or more fibers may be present in the composition at a level of 0.01-5 % by weight.

The composition can also include one or more hair active agents, such as straighteners, conditioners, hair growth agents, for example one or more of progesterones and progestamides, curling compounds, amino acids, proteins, carageenen, algae extract, wheat flour lipids, and dyes. In some aspects, the one or more hair active agents may be present in the composition at a level of 0.01-10 % by weight.

The composition can be a liquid at room temperature, and can include dispersed or dissolved particles.

The composition can be provided in a container, for example, a glass or plastic container, which can be, for example, a bottle, hard tube, squeezable tube, or pouch. The container can be provided with an applicator, such as a brush or pen applicator. The applicator can be integrated with one or more parts of the container. For example, a brush applicator may be integrated with the top of a bottle. The applicator can also be provided separately.

A method of using the composition can involve treating keratinous fibers by applying the composition to keratinous fibers. Keratinous fibers can include, for example, hair, such as human hair. The human hair can be, for example, hair of the head, eyelashes, or eyebrows.

The composition can be made by admixing the components, for example one or more of the components discussed above, in appropriate amounts and in appropriate sub-combinations before making the final product.

The following are exemplary compositions intended for use as a mascara, followed by a preferred manufacturing method.

### EXAMPLE 1

| ***Phase*** | ***Material Name*** | ***% Material*** | ***Quantity(g)*** |
|---|---|---|---|
| A | POLYBUTENE | 1.5000 | 5.2500 |
| A | VP/EICOSENE COPOLYMER | 1.5000 | 5.2500 |
| A | GLYCERYL STEARATE SE | 14.5000 | 50.7500 |
| A | HYDROGENATED OLIVE OIL STEARYL ESTERS | 6.0000 | 21.0000 |
| A | PEG-30 DIPOLYHYDROXYSTEARATE | 2.0000 | 7.0000 |
| A | CYCLOPENTASILOXANE | 7.0000 | 24.5000 |
| B | CI 77499 | 8.0000 | 28.0000 |
| B | ACACIA SENEGAL GUM | 2.0000 | 7.0000 |
| C | AQUA | 49.3000 | 172.5500 |
| C | HYDROXYETHYLCELLULOSE | 0.2000 | 0.7000 |
| C | POLYVINYL ALCOHOL | 0.5000 | 1.7500 |
| C | VP/VA COPOLYMER AQUA | 1.0000 | 3.5000 |
| C | PANTHENOL PROPYLENE GLYCOL | 0.5000 | 1.7500 |
| C | NYLON-12 | 1.5000 | 5.2500 |
| C | HYDRATED SILICA | 1.5000 | 5.2500 |
| C | PHENOXYETHANOL ETHYLHEXYLGLYCERIN | 1.0000 | 3.5000 |
| D | ALCOHOL DENAT. | 5.0000 | 17.5000 |
| **Total** | | **103.0000** | **360.5000** |

The table shows 103% material. This increased percentage is listed to account for water loss during manufacture.

### EXAMPLE 2

| **Phase** | **Material Name** | **% Material** |
|---|---|---|
| A | POLYBUTENE | 1.50 |
| A | GLYCERYL STEARATE SE | 13.50 |
| A | BEESWAX | 6.00 |
| A | PEG-30 DIPOLYHYDROXYSTEARATE | 3.00 |
| A | CYCLOPENTASILOXANE | 7.00 |
| B | BLACK IRON OXIDE | 8.00 |
| B | ACACIA SENEGAL GUM | 2.00 |
| C | WATER | 50.30 |
| C | HYDROXYETHYLCELLULOSE | 0.20 |
| C | VP/VA COPOLYMER | 1.00 |
| C | HYDRATED SILICA | 1.50 |
| C | PHENOXYETHANOL | 1.00 |
| D | ALCOHOL DENAT. | 5.00 |

### EXAMPLE 3

| **Phase** | **Material Name** | **% Material** |
|---|---|---|
| A | POLYBUTENE | 1.50 |
| A | POTASSIUM CETYL PHOSPATE | 6.00 |
| A | BEESWAX | 9.00 |
| A | PEG-30 DIPOLYHYDROXYSTEARATE | 3.00 |
| A | CYCLOPENTASILOXANE | 7.00 |
| B | BLACK IRON OXIDE | 8.00 |
| B | ACACIA SENEGAL GUM | 2.00 |
| C | WATER | 54.80 |
| C | HYDROXYETHYLCELLULOSE | 0.20 |
| C | VP/VA COPOLYMER | 1.00 |
| C | HYDRATED SILICA | 1.50 |
| C | PHENOXYETHANOL | 1.00 |
| D | ALCOHOL DENAT. | 5.00 |

### EXAMPLE 4

| **Phase** | **Material Name** | **% Material** |
|---|---|---|
| A | POLYBUTENE | 1.50 |
| A | SODIUM STEAROYL GLUTAMMATE | 3.00 |
| A | BEESWAX | 12.00 |
| A | PEG-30 DIPOLYHYDROXYSTEARATE | 3.00 |
| A | CYCLOPENTASILOXANE | 7.00 |
| B | BLACK IRON OXIDE | 8.00 |
| B | ACACIA SENEGAL GUM | 2.00 |
| C | WATER | 54.80 |
| C | HYDROXYETHYLCELLULOSE | 0.20 |
| C | VP/VA COPOLYMER | 1.00 |
| C | HYDRATED SILICA | 1.50 |
| C | PHENOXYETHANOL | 1.00 |
| D | ALCOHOL DENAT. | 5.00 |

### EXAMPLE 5

| **Phase** | **Material Name** | **% Material** |
|---|---|---|
| A | PEG -30 DIPOLYHYDROXYSTEARATE | 2.00 |
| A | CARNAUBA WAX | 10.00 |
| A | STEARIC ACID | 8.00 |
| B | ACACIA GUM | 8.00 |
| B | SILICA | 1.00 |
| B | BLACK IRON OXIDE | 10.00 |
| C | WATER | 58.30 |
| C | D- PANTENOL USP | 1.00 |
| C | PHENOXYETHANOL | 0.80 |
| C | ETHYLHEXYLGLYCERIN | 0.20 |
| C | SODIUM HYDROXIDE | 0.70 |

### EXAMPLE 6

| **Phase** | **Material Name** | **%** |
|---|---|---|
| A | PEG -30 DIPOLYHYDROXYSTEARATE | 2.00 |
| A | CARNAUBA WAX | 10.00 |
| A | STEARIC ACID | 8.00 |
| B | ACACIA GUM | 8.00 |
| B | SILICA | 1.00 |
| B | BLACK IRON OXIDE | 10.00 |
| C | WATER | 56.90 |
| C | D- PANTENOL USP | 1.00 |
| C | PHENOXYETHANOL | 0.80 |
| C | ETHYLHEXYLGLYCERIN | 0.20 |
| C | AMINO METHYL PROPANEDIOL | 2.10 |

### EXAMPLE 7

| **Phase** | **Material Name** | **%** |
|---|---|---|
| A | PEG -30 DIPOLYHYDROXYSTEARATE | 2.00 |
| A | CARNAUBA WAX | 10.00 |
| A | STEARIC ACID | 8.00 |
| B | ACACIA GUM | 8.00 |
| B | SILICA | 1.00 |
| B | BLACK IRON OXIDE | 10.00 |
| C | WATER | 58.50 |
| C | D- PANTENOL USP | 1.00 |
| C | PHENOXYETHANOL | 0.80 |
| C | ETHYLHEXYLGLYCERIN | 0.20 |
| C | AMMONIA | 0.50 |

The exemplary compositions may be prepared by the following manufacturing method:

Phase A and Phase B are heated separately to about 85-90 °C. Phase B is then added into Phase A, and the combination is heated at about 85 °C for about 30 mins. Phase C is separately homogenized for about 15 mins at about 85 °C. Once homogenized, Phase C is added into the combination of Phase A and Phase B, and the mixture of the three phases is further homogenized for about 15 mins at about 85 °C. In some aspects, cyclopentasiloxane may be added to the mixture at the end of the about 15 mins.

The mixture may then be cooled down to about 60 °C. In some aspects, the mixture may then be combined and mixed well with Phase D.

The mixture may then be cooled down to about 40 °C. In some aspects, the mixture may be cooled down to about 40 °C using a cold water bath, or other suitable means as will be known to those of ordinary skill in the art. The mixture may then be stored overnight at about 25 °C to about 30 °C. In some aspects, if paraffin is present in Phase A, the cool-down process is preferably conducted slowly (i.e., not shock freezing). Without wishing to be bound to any particular theory, it is believed that a slow cool-down process for paraffin-containing Phase A prevents the materials from hardening at the edge inside of the vessel, thus providing a smoother texture to the finished product.

As used herein, the term "about" is defined as being close to as understood by one of ordinary skill in the art. In one non-limiting embodiment, the term "about" is defined to be within 10%, preferably within 5%, more preferably within 1%, and most preferably within 0.5%.

While the aspects described herein have been described in conjunction with the example aspects outlined above, various alternatives, modifications, variations, improvements, and/or substantial equivalents, whether known or that are or may be presently unforeseen, may become apparent to those having at least ordinary skill in the art. Accordingly, the example aspects, as set forth above, are intended to be illustrative, not limiting. Various changes may be made without departing from the spirit and scope of the disclosure. Therefore, the disclosure is intended to embrace all known or later-developed alternatives, modifications, variations, improvements, and/or substantial equivalents.

Thus, the claims are not intended to be limited to the aspects shown herein, but are to be accorded the full scope consistent with the language of the claims, wherein reference to an element in the singular is not intended to mean "one and only one" unless specifically so stated, but rather "one or more." All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed as a means plus function unless the element is expressly recited using the phrase "means for."

Further, the word "example" is used herein to mean "serving as an example, instance, or illustration." Any aspect described herein as "example" is not necessarily to be construed as preferred or advantageous over other aspects. Unless specifically stated otherwise, the term "some" refers to one or more. Combinations such as "at least one of A, B, or C," "at least one of A, B, and C," and "A, B, C, or any combination thereof" include any combination of A, B, and/or C, and may include multiples of A, multiples of B, or multiples of C. Specifically, combinations such as "at least one of A, B, or C," "at least one of A, B, and C," and "A, B, C, or any combination thereof" may be A only, B only, C only, A and B, A and C, B and C, or A and B and C, where any such combinations may contain one or more member or members of A, B, or C. Nothing disclosed herein is intended to be dedicated to the public regardless of whether such disclosure is explicitly recited in the claims.

Moreover, all references throughout this application, for example patent documents including issued or granted patents or equivalents; patent application publications; and non-patent literature documents or other source material; are hereby incorporated by reference herein in their entireties, as though individually incorporated by reference.

## Claims

1. A cosmetic composition comprising:
a wax-in-water emulsion;
a water-in-oil co-emulsifier present at 1-20% by weight; and
amino methyl propanediol,
wherein the composition does not contain triethanolamine.

2. The cosmetic composition of claim 1, wherein the water-in-oil co-emulsifier is polyglyceryl-3-rice branate, cetearyl alcohol, polyglyceryl-10 stearate, polyglyceryl-6 stearate, hydroxypropyl guar, cetearyl alcohol, glyceryl stearate, PEG-30 dipolyhydroxystearate, PEG-40 stearate, cetearet-20, 165-PEG-100 stearate, glyceyl stearate, or a combination of the foregoing.

3. The cosmetic composition of claim 1 or 2, further comprising one or more ingredients selected from the group consisting of waxes, gums, cellulose polymers, non-volatile oils, volatile oils, water, alcohols, humectants, fillers, pigments, colorants, preservatives, film-forming agents, structuring agents, fragrances, fibers, and hair active agents.

4. The cosmetic composition of claim 1, wherein the composition is formulated as a mascara.

5. The cosmetic composition of claim 1, wherein the composition is formulated as a hair dye, conditioner, shampoo, or a combination thereof.

6. The cosmetic composition of claim 1, 2 or 3, wherein the composition comprises:
one or more of each of the following ingredients:
gums,
preservatives, and
fillers, pigments, and colorants; and
water.

7. The cosmetic composition of claim 6, wherein the composition further comprises fibers.

8. A method of preparing a cosmetic composition, the method comprising:
admixing a wax-in-water emulsion with a water-in-oil co-emulsifier and amino methyl propanediol, wherein the water-in-oil co-emulsifier is present at 1-20% by weight, to obtain a cosmetic composition that does not contain triethanolamine.

9. The method of claim 8, wherein the water-in-oil co-emulsifier is polyglyceryl-3-rice branate, cetearyl alcohol, polyglyceryl-10 stearate, polyglyceryl-6 stearate, hydroxypropyl guar, cetearyl alcohol, glyceryl stearate, PEG-40 stearate, cetearet-20, 165-PEG-100 stearate, glyceyl stearate, or a combination of the foregoing.

10. The method of claim 8 or 9, wherein the wax-in-water emulsion is admixed with the water-in-oil co-emulsifier and one or more ingredients selected from the group consisting of waxes, gums, cellulose polymers, non-volatile oils, volatile oils, water, alcohols, humectants, fillers, pigments, colorants, preservatives, film-forming agents, structuring agents, fragrances, fibers, and hair active agents.

11. The method of claim 8, 9 or 10, wherein the cosmetic composition comprises:
one or more of each of the following ingredients:
gums,
preservatives, and
fillers, pigments, and colorants; and
water.

12. The method of claim 11, wherein the cosmetic composition further comprises fibers.
